# FASCICULE DE BREVET EUROPEEN

(11) **EP 3 313 823 B1**
(45) Date de publication et mention de la délivrance du brevet: **09.11.2022**
(21) Numéro de dépôt: 16734229.4
(22) Date de dépôt: 23.06.2016
(51) Int. Cl.: C07D 233/34

(54) **PROCÉDÉ DE SYNTHÈSE DE MOLÉCULES PORTANT UNE FONCTION OXYDE DE NITRILE**
VERFAHREN ZUR SYNTHESE VON MOLEKÜLEN MIT NITRILOXIDFUNKTION
METHOD FOR SYNTHESIZING MOLECULES HAVING A NITRILE OXIDE FUNCTION

(30) Priorité: 24.06.2015 FR 1555822
(43) Date de publication de la demande: 02.05.2018
(73) Titulaire: Compagnie Générale des Etablissements Michelin, 63000 Clermont-Ferrand (FR); Arkema France, 92700 Colombes (FR)
(72) Inventeur: COUTURIER, Jean-Luc, 69006 Lyon (FR); LEDUC, Philippe, 69590 Larajasse (FR); IVANOV, Sergey, 63040 Clermont-Ferrand Cedex 09 (FR); UGOLNIKOV, Oleg, 63040 Clermont-Ferrand Cedex 09 (FR)
(74) Mandataire: Casalonga
(86) Numéro de dépôt international: PCT/EP2016/064493
(87) Numéro de publication internationale: WO 2016/207263

(56) Documents cités:
- WO-A1-2012/007684
- CN-A- 101 270 071

## Description

La présente invention concerne le domaine des molécules associatives azotées comprenant au moins un motif les rendant susceptibles de s'associer entre elles ou avec une charge par des liaisons non covalentes, et comprenant une fonction capable de réagir avec un polymère comprenant des insaturations pour former une liaison covalente avec ledit polymère.

Plus précisément, la présente invention concerne les molécules porteuses d'une fonction oxyde de nitrile et d'un groupe imidazolidinyle.

Plus précisément encore, la demande concerne un procédé de synthèse de telles molécules.

Dans le domaine industriel, des mélanges de polymères avec des charges sont souvent utilisés. Pour que de tels mélanges présentent de bonnes propriétés, on recherche en permanence des moyens pour améliorer la dispersion des charges au sein des polymères. L'un des moyens pour parvenir à ce résultat est l'utilisation d'agents de couplage capables d'établir des interactions entre le polymère et la charge.

Des agents de couplage d'un polymère avec une charge comprenant des dipôles azotés sont décrits dans les documents publiés sous les numéros US7186845B2 et JP2008208163.

Ces documents décrivent la modification de polymères comprenant des motifs diéniques par des composés dipolaires azotés comprenant en outre un hétérocycle, ledit hétérocycle comprenant luimême un atome d'azote, et un atome d'oxygène et/ou de soufre.

Plus particulièrement, les composés décrits sont des nitrones porteuses de fonction oxazoline, thiazoline comme par exemple la ((2-oxazolyl)-phényl-N-méthylnitrone).

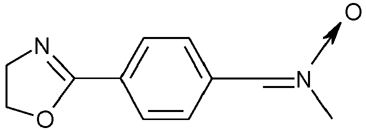

Lorsque des polymères diéniques sont amenés à réagir avec de tels composés, les polymères en résultant porteront les cycles oxazoline ou thiazoline.

Ces cycles présents sur le polymère sont susceptibles de réagir à leur tour, avec des fonctions de surface des charges, comme le noir de carbone ou la silice, avec lesquels les polymères sont mélangés. Cette réaction conduit à l'établissement de liaisons covalentes entre le polymère modifié par l'agent de couplage et la charge du fait de l'ouverture du cycle oxazoline ou thiazoline. En effet, comme cela est décrit dans le document US7186845B2, les cycles oxazolines et/ou thioazolines sont susceptibles de s'ouvrir en présence d'un nucléophile pouvant par exemple être présent à la surface de la charge.

L'établissement de telles liaisons covalentes présente néanmoins des inconvénients lors de la préparation de mélanges comprenant ces polymères modifiés par des agents de couplage avec des charges. En particulier, l'existence de ces liaisons covalentes précocement établies, entre le polymère et les charges, rend ces mélanges très visqueux à l'état non réticulé, ce qui rend difficile toutes les opérations préalables à la réticulation (vulcanisation) des formulations à base de caoutchouc, notamment la préparation des mélanges des composants, et leur mise en forme. Ces inconvénients ont un fort impact sur la productivité industrielle.

Il est donc souhaitable de proposer de nouvelles molécules ne présentant pas les inconvénients ci-dessus, c'est-à-dire des molécules qui soient capables après réaction avec un polymère et mélange avec une charge, de ne pas former de liaisons covalentes avec la charge et donc de ne pas provoquer une trop forte augmentation de la viscosité du mélange.

Ainsi, la demande de brevet WO 2012/007684 porte sur un composé comprenant au moins un groupement Q, et au moins un groupement A reliés entre eux par au moins et de préférence un groupe espaceur Sp dans lequel :
- Q comprend un dipôle contenant au moins et de préférence un atome d'azote ;
- A comprend un groupe associatif comprenant au moins un atome d'azote ;
- Sp est un atome ou un groupe d'atomes formant une liaison entre Q et A.

Lorsqu'un polymère greffé par un composé tel que défini ci-dessus est mélangé à des charges, celui-ci n'établit que des liaisons labiles avec les charges, ce qui permet d'assurer une bonne interaction polymère - charge, bénéfique pour les propriétés finales du polymère, mais sans les inconvénients qu'une trop forte interaction polymère - charge pourrait provoquer.

Un exemple d'un tel composé est l'oxyde de 2-[2-(2-oxoimidazolidin-1-léthox]benzonitrile :

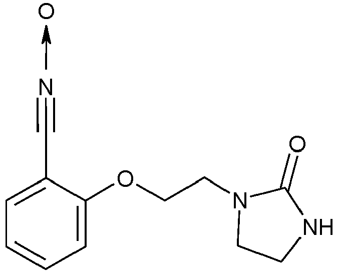

Ce composé peut être préparé à partir de l'aldéhyde salicylique et de la 1-(2-chloroéthyl)imidazolidin-2-one.

Dans une première étape, la 1-(2-chloroéthyl)imidazolidin-2-one est obtenue à partir de la 1-(2-hydroxyéthyl)imidazolidin-2-one qui réagit avec le chlorure de thionyle dans le dichlorométhane.

Dans une seconde étape, le 2-[2-(2-oxoimidazolidin-1-yl)éthoxy]benzaldéhyde est obtenu à partir de la 1-(2-chloroéthyl)imidazolidin-2-one qui réagit avec l'aldéhyde salicylique dans le diméthylformamide (DMF) en présence de carbonate de potassium.

Dans une troisième étape, la 2-[2-(2-oxoimidazolidin-1-yl)éthoxy]benzaldéhyde oxime est obtenue par mise en réaction du 2-[2-(2-oxoimidazolidin-1-yl)éthoxy]benzaldéhyde avec l'hydroxylamine dans l'éthanol.

Enfin, dans une quatrième étape, l'oxyde de 2-[2-(2-oxoimidazolidin-1-yl)éthoxy]benzonitrile est obtenu par mise en réaction de la 2-[2-(2-oxoimidazolidin-1-yl)éthoxy]benzaldéhyde oxime avec de l'hypochlorite de sodium dans le dichlorométhane.

Ainsi, le procédé décrit ci-dessus comprend un nombre d'étapes relativement important. La multiplication des étapes a un impact sur le rendement global du procédé de synthèse du composé désiré mais aussi sur les coûts d'investissement et les coûts de production associés. Par ailleurs, une autre préoccupation dans la synthèse des molécules porteuses d'une fonction oxyde de nitrile est de minimiser le nombre et la quantité de solvants utilisés dans le procédé de préparation, afin de faciliter le traitement des effluents, de réduire les coûts d'investissement et de production associés, bien sûr sans affecter ni le rendement, ni la pureté desdites molécules.

La présente invention a pour objet de proposer une solution permettant de résoudre l'ensemble des problèmes mentionnés ci-dessus.

Selon l'invention, un procédé de synthèse d'un composé de formule (I) : 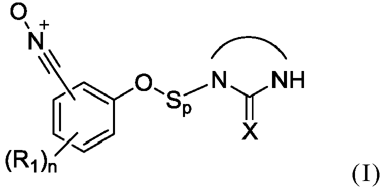 dans laquelle :
- X désigne un atome d'oxygène, de soufre ou un groupe NH, préférentiellement un atome d'oxygène ;
- R₁ représente un groupe alkyle en C₁-C₁₂ ou OR', R' étant un groupe alkyle en C₁-C₁₂ ;
- n est un nombre entier allant de 0 à 4 ;
- Sp représente une chaîne alkylène linéaire ou ramifiée en Ci-C₂₄, éventuellement interrompue par un ou plusieurs atomes d'azote ou d'oxygène,
   le groupe 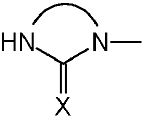 représentant un hétérocycle di ou triazoté, à 5 ou 6 atomes, de préférence diazoté, comprenant au moins une fonction carbonyle, thiocarbonyle ou imine,
   comprend deux étapes successives (b1) et (b2) selon une synthèse monotope :
      - (b1) la réaction d'un composé de formule (II) : 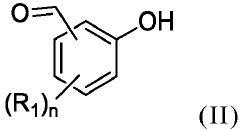 avec un composé de formule (III) : 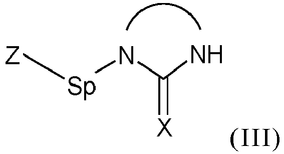 dans laquelle Z représente un groupe nucléofuge ;
   en présence :
      - d'au moins un solvant polaire S₁ ;
      - d'au moins une base choisie parmi les carbonates alcalins ;
   à une température T₁ allant de 70 à 150°C,
   pour former un éther ;
- (b2) la réaction dudit éther avec une solution aqueuse d'hydroxylamine à une température T₂ allant de 30 à 70°C, pour obtenir un composé oxime de formule (IV) : 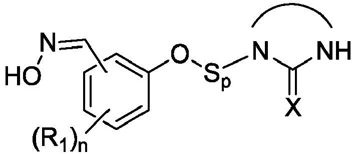 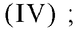 puis :
- une étape (c) de récupération dudit composé oxime de formule (IV) ;
- une étape (d) d'oxydation du composé oxime de formule (IV) avec un agent oxydant, en présence d'au moins un solvant organique S₂.

Les étapes (b1) et (b2) sont « one pot » (procédé de synthèse monotope en deux étapes), c'est-à-dire sans isolation de l'éther intermédiaire.

On entend par solvant polaire au sens de la présente invention un solvant présentant une constante diélectrique supérieure à 2,2.

De préférence, l'étape (b2) est suivie d'une étape (e) de récupération du composé de formule (I).

On entend par groupe nucléofuge au sens de la présente invention un groupe partant qui emporte son doublet de liaison.

On entend par chaîne carbonée au sens de la présente invention une chaîne comprenant un ou plusieurs atomes de carbone.

Il est précisé que les expressions « de... à... » utilisées dans la présente description doivent s'entendre comme incluant chacune des bornes mentionnées.

Le procédé selon l'invention permet de synthétiser le composé de formule (I) à partir du composé de formule (II) selon une synthèse monotope en deux étapes suivie d'une étape d'oxydation. Selon la demande de brevet WO 2012/007684, le composé de formule (I) est également obtenu à partir du composé de formule (II) mais le composé intermédiaire éther est isolé et purifié à l'issue de la première étape.

D'autres avantages et caractéristiques de l'invention apparaitront plus clairement à l'examen de la description détaillée.

Le procédé selon l'invention permet la synthèse du composé de formule (I) mentionnée ci-dessus.

Selon un mode de réalisation particulier de l'invention, le groupe 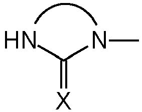 est le groupe 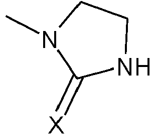 , dans lequel X est choisi parmi un atome d'oxygène et un atome de soufre, de préférence un atome d'oxygène.

Le groupe Sp est un groupe espaceur qui permet de relier l'atome d'oxygène au groupe 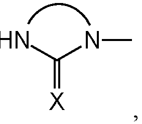 dans lequel X est tel que défini précédemment, tel que la formule du composé (I) l'indique.

On entend par fonctions associatives au sens de la présente invention, des fonctions susceptibles de s'associer les unes aux autres par des liaisons hydrogène.

Ainsi, chaque fonction associative comporte au moins un « site » donneur et un site accepteur vis-à-vis de la liaison hydrogène de sorte que deux fonctions associatives identiques sont auto-complémentaires et peuvent s'associer entre elles en formant au moins deux liaisons hydrogène.

Le groupe Sp est une chaîne alkylène linéaire ou ramifiée en C₁-C₂₄, préférentiellement en C₁-C₁₀, éventuellement interrompue par un ou plusieurs atomes d'azote ou d'oxygène, plus préférentiellement une chaîne alkylène linéaire en C₁-C₆.

De préférence, le groupe Sp contient un motif choisi parmi - (CH₂)_{y1}-, -[NH-(CH₂)_{y2}]ₓ₁- et -[O-(CH₂)_{y3}]ₓ₂-, y₁, y₂ et y₃ représentant, indépendamment, un nombre entier allant de 1 à 6, et x₁ et x₂ représentant, indépendamment, un nombre entier allant de 1 à 4.

R₁ représente un groupe alkyle en C₁-C₁₂, plus préférentiellement en C₁-C₆, encore plus préférentiellement en C₁-C₄, ou OR', R' étant un groupe alkyle en C₁-C₁₂, plus préférentiellement en C₁-C₆, encore plus préférentiellement en C₁-C₄, encore plus préférentiellement un groupe méthyle, éthyle ou un groupe OCH₃.

Avantageusement, n est un nombre entier allant de 0 à 3.

Préférentiellement, le composé de formule (I) est choisi parmi les composés de formule (V), (VI) et (VII) suivantes :

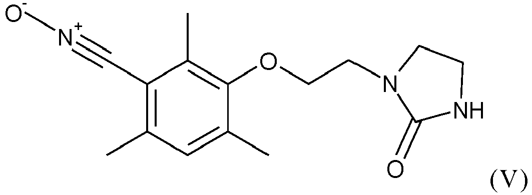

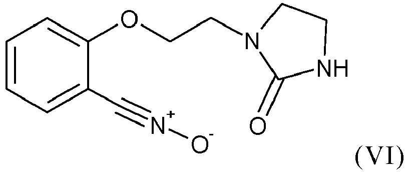

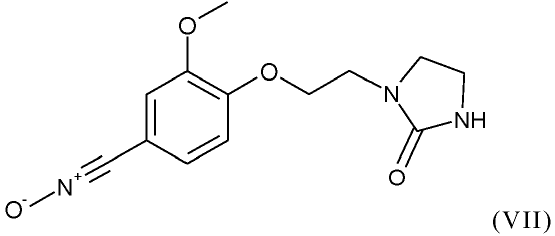

Comme expliqué précédemment, le procédé selon l'invention comprend une étape (b1) de réaction d'un composé de formule (II), tel que mentionnée ci-dessus, avec un composé de formule (III), telle que mentionnée ci-dessus, porteur d'un groupe Z.

De manière préférée, le groupe Z est choisi parmi le chlore, le brome, l'iode, le groupe mésylate, le groupe tosylate, le groupe acétate et le groupe trifluorométhylsulfonate.

Ladite étape (b1) du procédé selon l'invention est réalisée en présence d'au moins un solvant polaire Si, et d'au moins une base choisie parmi les carbonates alcalins, à une température T₁ allant de 70 à 150°C.

Selon un mode de réalisation particulier de l'invention, le solvant polaire S₁ est un solvant polaire miscible dans l'eau, préférentiellement un solvant protique.

Le diméthylformamide (DMF), le diméthylsulfoxyde (DMSO), la 1,3-diméthyl-2-imidazolidinone (DMI), la 1,3-diméthyl-3,4,5,6-tetrahydro-2(1H)-pyrimidinone (DMPU), l'isopropanol, l'acétonitrile, l'éthanol, le n-butanol et le n-propanol sont des exemples de solvants utilisables dans le procédé selon l'invention.

De préférence, le solvant protique est alcoolique.

Avantageusement, le composé de formule (II) représente de 5 à 40% en poids, de préférence de 10 à 30% en poids, par rapport au poids du solvant.

Avantageusement, il est possible d'ajouter :
- un ou plusieurs catalyseurs choisis parmi un catalyseur de type sel d'argent (I), un catalyseur de transfert de phase de type ammonium quaternaire, et leurs mélanges ;
- un ou plusieurs liquides ioniques.

Préférentiellement, la base est choisie parmi le carbonate de potassium.

Selon un mode de réalisation particulier de l'invention, la quantité de base est de 1,5 à 8 équivalents, de préférence de 2 à 6 équivalents, par rapport à la quantité de composé de formule (II).

Comme expliqué précédemment, l'étape (b1) du procédé selon l'invention est réalisée à une température T₁ allant de 70 à 150°C.

De préférence, la température T₁ est une température allant de 70 à 120°C, plus préférentiellement de 80 à 110°C.

Comme expliqué précédemment, l'étape (b1) du procédé selon l'invention est suivie de l'étape (b2) de l'ajout dans le milieu réactionnel contenant ledit éther d'une solution aqueuse d'hydroxylamine à une température T₂ allant de 30 à 70°C.

Préférentiellement, l'ajout de la solution aqueuse d'hydroxylamine est réalisé lorsque la conversion du composé de formule (II) est d'au moins 70%.

Avantageusement, la température T₂ varie de 40 à 60°C.

Le procédé selon l'invention comprend également une étape (c) de récupération, telle que mentionnée ci-avant, du composé oxime de formule (IV).

De manière préférée, le composé oxime de formule (IV) est récupéré par précipitation à l'eau, suivie éventuellement d'un lavage à l'eau.

Le procédé selon l'invention comprend également une étape (d) d'oxydation du composé oxime de formule (IV) avec un agent oxydant, en présence d'au moins un solvant organique S₂.

De manière préférée, ledit agent oxydant est choisi parmi l'hypochlorite de sodium, le N-bromosuccinimide en présence d'une base, le N-chlorosuccinimide en présence d'une base, et l'eau oxygénée en présence d'un catalyseur, préférentiellement l'hypochlorite de sodium.

Avantageusement, la quantité d'agent oxydant est de 1 à 5 équivalents, préférentiellement de 1 à 2 équivalents, par rapport à la quantité de composé oxime de formule (IV).

Préférentiellement, le solvant organique S₂ est un solvant organique choisi parmi les solvants chlorés et de type ester, éther et alcool, plus préférentiellement choisi parmi le dichlorométhane, l'acétate d'éthyle, l'acétate de butyle, l'éther diéthylique, l'isopropanol et l'éthanol, encore plus préférentiellement choisi parmi l'acétate d'éthyle et l'acétate de butyle.

De préférence, le composé oxime de formule (IV) représente de 1 à 30% en poids, de préférence de 1 à 20% en poids, par rapport au poids total de l'ensemble comprenant ledit composé oxime de formule (IV), ledit solvant organique S₂ et ledit agent oxydant.

Préférentiellement, le procédé selon l'invention comprend une étape (e) de récupération, telle que mentionnée ci-avant, du composé de formule (I).

De manière préférée, le composé de formule (I) est récupéré par précipitation à l'eau, suivie éventuellement d'un lavage à l'eau.

De préférence, le procédé selon l'invention comprend une étape (a1) de fabrication du composé de formule (II), préalable à l'étape (b1), par mise en réaction d'un composé de formule (VIII) : 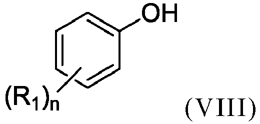 avec un agent de formylation formé in situ ou non et au moins un acide de Lewis, en présence d'au moins un solvant organique S₃.

De manière préférée, l'acide de Lewis est choisi parmi TiCl₄ et SnCl₄, préférentiellement TiCl₄.

De préférence, la quantité d'acide de Lewis est de 0,5 à 4 équivalents, plus préférentiellement de 1 à 3 équivalents, par rapport à la quantité de composé de formule (VIII).

Avantageusement, le solvant organique S₃ est un solvant organique chloré, préférentiellement choisi parmi le dichlorométhane, le chloroforme et le 1,2-dichloroéthane, plus préférentiellement le dichlorométhane.

Comme expliqué précédemment, le composé de formule (VIII) réagit avec un agent de formylation formé in situ ou non, selon une réaction de formylation. On peut utiliser tout type d'agent de formylation classiquement employé dans les réactions de formylation.

Préférentiellement, l'agent de formylation est choisi parmi le dichlorométhyl-méthyléther et le dichlorométhyl-éthyléther, plus préférentiellement le dichlorométhyl-méthyléther.

Selon un mode de réalisation particulier de l'invention, l'agent formé in situ est obtenu par réaction du formiate de méthyle ou d'éthyle avec un agent de chloration, préférentiellement le pentachlorure de phosphore.

Avantageusement, le composé de formule (VIII) représente de 5 à 15% en poids par rapport au poids du solvant.

Selon un mode de réalisation particulier de l'invention, le procédé selon l'invention comprend une étape (a2) de fabrication du composé de formule (III), préalable à l'étape (b1), par mise en réaction d'un composé de formule (IX) : 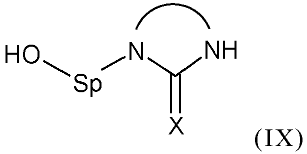 avec un agent permettant la formation du groupe nucléofuge Z.

De manière préférée, ledit agent est le chlorure de thionyle.

De préférence, l'étape (a2) est réalisée en l'absence ou en présence d'au moins un solvant, préférentiellement un solvant chloré, plus préférentiellement le dichlorométhane.

Avantageusement, l'étape (a2) est immédiatement suivie d'une étape (a3) de récupération du composé de formule (III), préférentiellement par purification au toluène, plus préférentiellement par cristallisation du composé de formule (III) dans le toluène.

La présente invention est en outre illustrée par les exemples non limitatifs suivants.

### EXEMPLES

L'analyse structurale ainsi que la détermination des puretés molaires des molécules de synthèse sont réalisées par une analyse RMN. Les spectres sont acquis sur un spectromètre Avance 500 MHz BRUKER équipé d'une sonde "large bande" BBIz-grad 5 mm. L'expérience RMN ¹H quantitative, utilise une séquence simple impulsion 30° et un délai de répétition de 3 secondes entre chacune des 64 acquisitions. Les échantillons sont solubilisés dans le diméthylsulfoxide deutéré (DMSO). Ce solvant est également utilisé pour le signal de lock. La calibration est réalisée sur le signal des protons du DMSO deutéré à 2,44 ppm par rapport à une référence TMS à 0 ppm. Le spectre RMN ¹H couplé aux expériences 2D HSQC ¹H/¹³C et HMBC ¹H/¹³C permettent la détermination structurale des molécules (cf tableaux d'attributions). Les quantifications molaires sont réalisées à partir du spectre RMN 1D ¹H quantitatif.

L'analyse par spectrométrie de masse est réalisée en injection directe par un mode d'ionisation en electrospray (ID/ESI). Les analyses ont été réalisées sur un spectromètre HCT Bruker (débit 600 µL/min, Pression du gaz nébuliseur 10 psi, débit du gaz nébuliseur 4 L/min).

### Exemple 1 : Synthèse de l'oxyde de 2-[2-(2-oxoimidazolidin-1-yl)éthoxy]benzonitrile

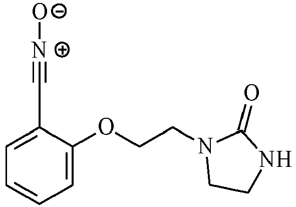

L'oxyde de 2-[2-(2-oxoimidazolidin-1-yl)éthoxy]benzonitrile est synthétisé à partir du saliçaldéhyde et de la 1-(2-chloroéthyl)imidazolidin-2-one grâce à une synthèse monotope en deux étapes b1 et b2 (suivie d'une étape de récupération c) suivie d'une étape d'oxydation d. Une oxime peut être tout d'abord préparée indépendamment selon les deux protocoles 1 et 2 indiqués ci-dessous avant la réalisation d'une étape d'oxydation, selon le protocole 3, afin d'obtenir ledit oxyde.

### Synthèse monotope en deux étapes b1 et b2 suivie de l'étape c (conforme à l'invention) : préparation de l'oxime de 2-(2-(2-oxoimidazolidin-1-yl)éthoxy) benzaldéhyde

Protocole 1 (selon l'invention) :
Dans un réacteur de 1L, on charge 30 g de saliçaldéhyde (0,25 mol) et 200 g d'éthanol. On ajoute 172,8 g de carbonate de potassium (1,25 mol) et on chauffe à reflux. On ajoute ensuite, par portions et sur une période de 5 heures, 92,8 g de 1-(2-chloroéthyl)imidazolidin-2-one (0,63 mol). A la fin de l'ajout, on laisse réagir pendant 2 heures à reflux. On refroidit à 50°C, puis on ajoute, sur une période de 15 minutes, 24,8 g d'une solution aqueuse d'hydroxylamine à 50% (0,38 mol). On laisse réagir pendant 2 heures à 50°C. Le mélange réactionnel est concentré jusqu'à un volume de 50 mL, puis on ajoute, à température ambiante, 500 mL d'eau. Le précipité obtenu est filtré, lavé à l'eau puis au pentane, et séché sous vide.

Un solide blanc (40,5 g, rendement massique de 65%) avec un point de fusion de 88°C est obtenu.

Protocole 2 (selon l'invention) :
Dans un réacteur de 1L, on charge 30 g de saliçaldéhyde (0,25 mol) et 300 g d'acétonitrile. On ajoute 172,8 g de carbonate de potassium (1,25 mol) et on chauffe à reflux. On ajoute ensuite, par portions et sur une période de 6 heures, 109,5 g de 1-(2-chloroéthyl)imidazolidin-2-one (0,74 mol). A la fin de l'ajout, on laisse réagir pendant 2 heures à reflux. On refroidit à 50°C, puis on ajoute, sur une période de 15 minutes, 24,8 g d'une solution aqueuse d'hydroxylamine à 50% (0,38 mol). On laisse réagir pendant 2 heures à 50°C. Le mélange réactionnel est concentré, puis on ajoute, à température ambiante, 500 mL d'eau. Le précipité obtenu est filtré, lavé à l'eau puis au pentane, et séché sous vide.

Un solide blanc (41,8 g, rendement massique de 70%) avec un point de fusion de 88°C est obtenu.

### Etape d: préparation de l'oxyde de 2-[2-(2-oxoimidazolidin-1-yl)éthoxy]benzonitrile

Protocole 3 :
Dans un réacteur de 1L, on charge 30 g d'oxime de 2-(2-(2-oxoimidazolidin-1-yl)éthoxy)benzaldéhyde (0,12 mol) et 300 g d'acétate d'éthyle. On refroidit à 5°C et on ajoute, sur une période de 15 minutes, 111,7 g d'eau de Javel à 12% (0,18 mol). On laisse réagir pendant 3 heures à 5°C. On filtre et on lave le solide obtenu à l'eau puis à l'acétate d'éthyle.

Un solide blanc-jaune (17,8 g, rendement massique de 60%) avec un point de fusion de 109°C est obtenu.

### Exemple 2 : Synthèse de l'oxyde de 2,4,6-triméthyl-3-[2-(2-oxoimidazolidin-1-yl)éthoxy] benzonitrile

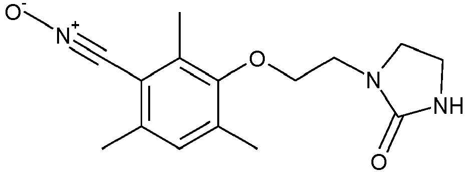

L'oxyde de 2,4,6-triméthyl-3-[2-(2-oxoimidazolidin-1-yl)éthoxy]benzonitrile est préparé selon deux voies de synthèse. Selon une première voie, ledit oxyde est synthétisé en six étapes, nommées étape a1, étape a2, étape b1, étape b2, étape c et étape d. La seconde voie de synthèse diffère de la première voie en ce que les étapes b1 et b2 sont réalisées selon une synthèse monotope en deux étapes.

Pour chacune des étapes, les composés peuvent être synthétisés indépendamment selon plusieurs protocoles. L'étape a1 est réalisée selon divers protocoles 4 à 9, l'étape a2 selon divers protocoles 10 à 12, l'étape b1 selon divers protocoles 13 à 15. Les étapes b2 et c sont réalisées selon deux protocoles 16 et 17.

La synthèse monotope en deux étapes b1 et b2 suivie de l'étape c sont réalisées selon divers protocoles 18 à 21.

L'étape d est réalisée selon divers protocoles 22 à 24.

### Etape a1 : préparation du 3-hydroxy-2,4,6-triméthylbenzaldéhyde

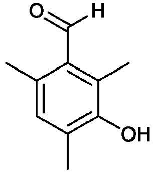

Protocole 4 :
Ce composé peut être obtenu selon un protocole décrit dans la demande de brevet WO 2012/007684.

Protocole 5 :
A une solution de TiCl₄ (10,44 g, 0,055 mol) dans le dichlorométhane (5 mL) refroidi à 12 °C, est ajoutée pendant 10 minutes une solution de mésitol (5,0 g, 0,037 mol) dans le dichlorométhane (15 mL). Après agitation pendant 5-10 minutes à 10-15°C, une solution de dichlorométhylméthyl éther (6,75 g, 0,059 mol) dans le dichlorométhane (5 mL) est ajoutée sur une période de 10-15 minutes. Après 15 heures à température ambiante, un mélange de 100 mL d'eau et 50 g de glace est versé dans le milieu réactionnel. Après 30-40 minutes d'agitation, le brut réactionnel est filtré, lavé par l'eau (4 fois par 10 mL) et séché sous air.

Le produit cible (3,93 g) est obtenu avec un rendement massique de 65 %.

La pureté molaire estimée par RMN ¹H est supérieure à 90 %.

Chromatographie sur couches minces (CCM) : Rf = 0,87 (SiO₂ ; EtOAc ; révélation par UV et I₂), ou Rf = 0,35 (SiO₂ ; heptane : EtOAc = 3 : 1 ; révélation par UV et I₂).

Protocole 6 :
A une solution de mésitol (6,0 g, 0,044 mol) dans le dichlorométhane (30 mL) (sous atmosphère inerte) à 2°C, est ajouté goutte à goutte du TiCl₄ (15,04 g, 0,079 mol) sur 15-20 minutes en maintenant la température du milieu réactionnel inférieure à 8°C. Après 5 minutes d'agitation à 6°C, le dichlorométhylméthyl éther (7,60 g, 0,066 mol) est ajouté pendant 35-40 minutes. Après 16 heures à température ambiante, un mélange de 75 mL d'eau et 50 g de glace est versé dans le milieu réactionnel. Apres 1 heure et 30 minutes d'agitation, le produit est filtré, lavé par l'eau (4 fois par 5 mL), et séché sous air.

Le produit cible (5,53 g, 0,034 mol) est obtenu avec un rendement massique de 76 %.

La pureté molaire estimée par RMN ¹H est supérieure à 98 %.

CCM : Rf = 0,87 (SiO₂ ; EtOAc ; révélation par UV et I₂), ou Rf = 0,35 (SiO₂ ; heptane : EtOAc = 3 : 1 ; révélation par UV et I₂).

Protocole 7 :
A une solution de SnCl₄ (183,6 g, 0,705 mol) dans le dichlorométhane (250 mL) refroidie à 13°C, est ajoutée sur 20 minutes une solution de mésitol (40,0 g, 0,294 mol) dans le dichlorométhane (250 mL). Après 5 minutes d'agitation à 13°C, une solution de dichlorométhylméthyl éther (50,6 g, 0,441 mol) dans le dichlorométhane (100 mL) est ajoutée pendant 30 minutes. Le milieu réactionnel est agité pendant 15 heures à température ambiante.

Le milieu réactionnel est ensuite versé sur un mélange eau/glace (800 mL d'eau et 0,7 kg de glace). Après une heure d'agitation, la phase organique est séparée. La phase aqueuse est extraite par CH₂Cl₂ (2 fois par 100 mL). Les phases organiques réunies sont lavées par l'eau (2 fois par 100 mL), séchées par Na₂SO₄ et concentrées sous pression réduite (10 mbar, 23°C) pour conduire à 53 g d'une huile.

Une solution aqueuse de diméthyle amine (500 mL, 40% dans l'eau) est ajoutée au brut réactionnel. Le mélange est chauffé à 60°C et agité à cette température pendant 2,5 heures. De l'eau (1,0 L) est ajoutée et la phase aqueuse est extraite par le dichlorométhane (4 fois par 100 mL). Les phases organiques rassemblées sont lavées par une solution de HCl/eau (1 : 2) (trois fois par 100 mL) et par l'eau (trois fois par 100 mL). Après concentration sous pression réduite (150 mbar, 23°C), de l'éther de pétrole 40/60 (100 mL) est ajouté. La solution est refroidie jusqu'à -18°C pendant 15 heures. Les cristaux obtenus sont filtrés et lavés par un mélange de dichlorométhane (5 mL) et d'éther de pétrole 40/60 (15 mL), puis par de l'éther de pétrole 40/60 (deux fois par 20 mL), et enfin séchés sous air.

Le solide (20,02 g) est obtenu avec un rendement massique de 42 %.

Après concentration sous pression réduite du filtrat (60 mbar, 23°C), de l'éther de pétrole 40/60 (70 mL) y est ajouté. La solution est refroidie à -18 °C pendant 6 heures. Les cristaux obtenus sont filtrés sont lavés par l'éther de pétrole 40/60 (deux fois par 10 mL), et séchés sous air.

La seconde fraction de produit (3,02 g) est obtenue avec un rendement de 6,3 %.

Une purification par trituration est réalisée selon le protocole indiqué ci-dessous.

Les deux fractions de solides sont réunies et solubilisées dans un mélange de dichlorométhane (150 mL) et d'éther de pétrole 40/60 (100 mL). La solution est refroidie à -18°C pendant 4 à 6 heures. Les cristaux sont filtrés, lavés par l'éther de pétrole 40/60 (deux fois par 20 mL), et séchés sous air.

Le produit (11,9 g) est obtenu avec un rendement massique de 25 %.

La pureté molaire estimée par RMN ¹H est supérieure à 96 %.

CCM : Rf = 0,87 (SiO₂ ; EtOAc ; révélation par UV et I₂), ou Rf = 0,35 (SiO₂ ; heptane : EtOAc = 3 : 1 ; révélation par UV et I₂).

Protocole 8 :
A du PCl₅ (91,7 g, 0,441 mol), sous atmosphère inerte, est ajouté à 22-29°C pendant 70 minutes du formiate d'éthyle (34,8 g, 0,470 mol). La réaction étant exothermique, la température du milieu peut atteindre 40-60 °C. Le milieu réactionnel est agité pendant 1 heure à température ambiante. Une fois le PCl₅ totalement solubilisé, le milieu réactionnel est agité 1-2 heures supplémentaires à température ambiante. L'agent de formylation ainsi formé est utilisé dans l'étape suivante sans purification supplémentaire.

A une solution de mésitol (40,0 g, 0,294 mol) dans le dichlorométhane (100 mL) à 11°C, est ajouté du TiCl₄ (105,0 g, 0,529 mol) sur 45 minutes. Après 5 minutes à 12-13°C, l'agent de formylation précédemment préparé est additionné sur 1,5-2,0 heures en maintenant la température entre 9 et 22°C. La suspension obtenue est agitée pendant 15 heures à température ambiante. Le milieu réactionnel est ensuite versé sur un mélange d'eau (500 mL) et de glace (500 g). Apres 15-20 minutes d'agitation, le précipité est filtré et lavé par l'eau (5 fois par 50 mL). Les cristaux obtenus sont séchés sous air.

Le produit cible (27,5 g, 0,170 mol) est obtenu avec un rendement massique de 58 %.

La pureté molaire estimée par RMN ¹H : 97 % mol.

CCM : Rf = 0,35 (SiO₂ ; heptane : EtOAc = 3 : 1 ; révélation par UV et I₂).

Protocole 9 :
Dans un réacteur de 2 L muni d'un réfrigérant et purgé à l'azote, on charge 105,8 g de tétrachlorure de titane (0,56 mol). On refroidit à 15°C, et on coule, sur une période de 30 minutes, 40 g de mésitol (0,29 mol) dissous dans 400 g de dichlorométhane. En maintenant la température à 15-20°C, on coule ensuite, sur une période d'une heure, 40,6 g de dichlorométhyl-méthyléther (0,35 mol). A la fin de l'ajout, on laisse réagir pendant 3 heures à température ambiante. Le mélange réactionnel est transféré lentement dans un réacteur contenant 800 g d'eau et maintenu à une température inférieure à 20°C. Après 30 minutes sous agitation, on décante et on récupère la phase organique. La phase aqueuse est extraite deux fois avec 100 g de dichlorométhane. Les phases organiques rassemblées sont lavées avec 200 g d'eau, puis concentrées jusqu'à un volume de 100 mL. On rajoute 100 g de pentane, puis on refroidit à -15°C. Les cristaux obtenus sont lavés avec 50 g d'un mélange dichlorométhane-pentane 1/1 puis séchés sous vide.

Un solide blanc-beige (45,4 g, rendement massique de 94%) avec un point de fusion de 113°C est obtenu.

### Etape a2 : préparation de la 1-(2-chloroéthyl)imidazolidin-2-one

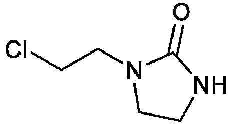

Protocole 10 :
Ce composé peut être obtenu selon un protocole décrit dans la demande de brevet WO 2012/007684.

Protocole 11 :
Sur de la 1-(2-hydroxyéthyl)imidazolidin-2-one (séché sous pression réduite à 60°C) (5,0 g, 38,42 mmol) est ajouté goutte à goutte du SOCl₂ (5,0 mL, 69,34 mmol) pendant 2-3 minutes. Le mélange est agité à 80°C (T_{bain}) pendant 1 heure et 30 minutes puis une heure et 30 minutes à 100°C (T_{bain}). Après évaporation de l'excès de SOCl₂ sous pression réduite (T_{bain} 50 °C, 8-9 mbar), le brut réactionnel (7,53 g, rendement 105,9 %) est obtenu sous la forme d'une huile jaune.

De l'eau (10 mL) est ajoutée au brut réactionnel et le pH de la phase aqueuse est ajusté à 8 par addition de carbonate de potassium.

Apres cristallisation à température ambiante dans l'eau, le précipité est filtré et lavé par l'eau (2-3 mL). Les cristaux sont séchés sous air.

Le produit cible (2,87 g, 19,31 mol) est obtenu avec un rendement massique de 50 %.

La pureté molaire estimée par RMN ¹H est supérieure à 90 %.

Protocole 12 :
120 g d'HEIO ((1-(2-hydroxyethyl)imidazolidin-2-one), 0,92 mol) sont chargés dans un réacteur de IL muni d'un réfrigérant et purgé à l'azote. On chauffe à 90°C, et 120,4 g de chlorure de thionyle (1,02 mol) sont ajoutés sur une période de 30 minutes. On laisse réagir 2 heures à 90°C. 400 g de toluène sont ensuite ajoutés et on chauffe à reflux pendant 2 heures. Le mélange réactionnel est refroidi à température ambiante. Le précipité obtenu est filtré, lavé au toluène, puis séché sous vide. On obtient 123,2 g d'un solide blanc (point de fusion égal à 93°C, rendement massique de 90%).

### Etape b1 (non conforme à l'invention) : préparation du 2,4,6-triméthyl-3-(2-(2-oxoimidazolidin-1-yl)éthoxy)benzaldéhyde

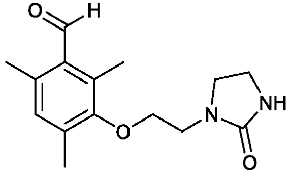

Protocole 13 :
Ce composé peut être obtenu selon un protocole décrit dans la demande de brevet WO 2012/007684.

Protocole 14 :
Un mélange de 3-hydroxy-2,4,6-triméthylbenzaldéhyde (25,0 g, 0,152 mol) et de K₂CO₃ (126,3 g, 0,914 mol) dans le DMF (200 mL) est agité à 30-35 °C pendant 5 à 10 minutes. Est ensuite ajoutée de la 1-(2-chloroéthyl)imidazolidin-2-one (33,9 g, 0,228 mol). La température du milieu réactionnel est portée à 90°C (T_{bain}) pendant 2,5 à 3 heures. Ensuite, une seconde portion de 1-(2-chloroéthyl)imidazolidin-2-one (22,6 g, 0,152 mol) est ajoutée et le mélange est porté à 100°C (T_{bain}) pendant 3 heures. Enfin, une troisième portion de 1-(2-chloroéthyl)imidazolidin-2-one (22,6 g, 0,152 mol.) est ajoutée et le mélange est porté à 110-115°C (T_{bain}) pendant 3 à 4 heures. Après un retour à 23°C, le milieu réactionnel est dilué par l'eau (4,0 L). La phase organique est extraite par du CH₂Cl₂ (6 fois par 200 mL). Les phases organiques rassemblées sont concentrées sous pression réduite (85 mbar, 36°C). Le résidu obtenu est repris par Et₂O (200 mL) et le mélange est agité à température ambiante pendant 2 heures. Le précipité obtenu est filtré, lavé sur le filtre par Et₂O (25 mL) et séché à température ambiante.

Un solide blanc (31,93 g, rendement massique de 76 %) est obtenu.

La pureté molaire est supérieure à 82 % (RMN ¹H).

Protocole 15 :
A un mélange de 3-hydroxy-2,4,6-triméthylbenzaldéhyde (0,44 g, 3,3 mmol), de 1-(2-hydroxyéthyl)imidazolidin-2-one (0,64 g, 4,9 mmol) et de triphénylphosphine (1,31 g, 5,0 mmol) dans le THF (30 mL) à température ambiante, est ajoutée une solution de diisopropyl azodicarboxylate (1,01 g, 5,0 mmol) dans le THF (15 mL) pendant 5 à 10 minutes. Le milieu réactionnel est agité pendant 15 heures à température ambiante et de l'eau (15 mL) y est ensuite ajoutée. Les solvants sont concentrés sous pression réduite (28°C, 50 mbar). La phase aqueuse est extraite par l'acétate d'éthyle (trois fois par 25 mL). Les phases organiques rassemblées sont lavées par une solution aqueuse saturée en NaCl. Le solvant est concentré sous pression réduite jusqu'à environ 3 mL (30°C). Apres purification sur colonne chromatographique (Ø 1,5 cm, H 11 cm) (éluant : acétate d'éthyle : éthanol (10 : 1)). Le solvant est évaporé jusqu'à sec.

Un solide blanc (0,77 g, rendement massique de 84 %) est obtenu.

La pureté molaire est supérieure à 78 % (RMN ¹H).

### Etapes b2 et c (non conformes à l'invention) : préparation de l'oxime de 2,4, 6-triméthyl-3-(2-(2-oxoimidazolidin-1-yl)éthoxy)benzaldéhyde

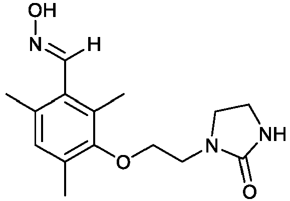

Les produits isolés à l'issue des protocoles 13, 14 et 15 sont utilisés dans les protocoles suivants (16, 17). L'enchainement de ces étapes est non conforme à l'invention du fait de l'isolation de ces produits.

Protocole 16 :
Ce composé peut être obtenu selon un protocole décrit dans la demande de brevet WO 2012/007684.

Protocole 17 :
A une solution de 2,4,6-triméthyl-3-(2-(2-oxoimidazolidin-1-yl)éthoxy)benzaldéhyde (31,5 g, 0,114 mol) dans l'EtOH (300 mL) à 44°C, est ajoutée une solution d'hydroxylamine (12,0 g, 0,182 mol., 50 % dans l'eau, Aldrich) dans l'EtOH (20 mL). Le milieu réactionnel est agité pendant 4 heures à 50 °C. De l'eau (80 mL) est ajoutée à la solution. Le milieu réactionnel et concentré sous pression réduite (80 mbar, 40°C). La solution obtenue est refroidie à 15-20°C et agitée pendant 5 à 10 minutes. Le précipité obtenu est filtré, lavé sur le filtre par un mélange éthanol/eau (deux fois par 10/15 mL) et séché pendant 15-20 heures sous pression atmosphérique à température ambiante.

Un solide blanc (28,32 g, rendement massique de 85 %) est obtenu.

La pureté molaire estimée par RMN ¹H est supérieure à 87 %.

CCM : Rf = 0,58 (SiO₂ ; EtOAc : EtOH = 5 : 1) ; révélation par UV et I₂).

### Synthèse monotope en deux étapes b1 et b2 suivie de l'étape c (conforme à l'invention) : préparation de l'oxime de 2,4,6-triméthyl-3-(2-(2-oxoimidazolidin-1-yl)éthoxy)benzaldéhyde

Protocole 18 (selon l'invention) :
A un mélange de 3-hydroxy-2,4,6-triméthyl-benzaldéhyde (10,0 g, 0,061 mol) et de K₂CO₃ (50,5 g, 0,365 mol) dans le DMF (100 mL) à 35°C, est ajoutée de la 1-(2-chloroéthyl)imidazolidin-2-one (13,55 g, 0,091 mol). La température du milieu réactionnel est portée à 90°C (T_{bain}) et celui-ci est agité à cette température pendant 2,5 à 3 heures. Ensuite, une seconde portion de 1-(2-chloroéthyl)imidazolidin-2-one (9,03 g, 0,061 mol) est ajoutée et le mélange est agité à 100°C (T_{bain}) pendant 2,5 heures. Enfin, la troisième portion de 1-(2-chloroéthyl)imidazolidin-2-one (9,03 g, 0,061 mol, pureté technique) est ajoutée et le mélange est agité à 105°C (Tbain) pendant 4 heures.

La température du milieu réactionnel est abaissée à 43°C, puis une solution d'hydroxylamine (6,84 g, 0,104 mol, 50 % dans l'eau, Aldrich) est ajoutée. Le milieu réactionnel est agité pendant 5 heures à 50-53°C. Une seconde portion de solution d'hydroxylamine (environ 3 g, 50 % dans l'eau, Aldrich) est ajoutée. Le milieu réactionnel est agité pendant 1,5 à 2 heures à 50-53 °C. Le mélange est alors dilué par de l'eau (1,25 L). La suspension obtenue est agitée pendant 15 heures à température ambiante. Le précipité obtenu est filtré, lavé sur le filtre par l'eau (4 fois par 50 mL) et séché à température ambiante.

Un solide gris clair (12,87 g, rendement massique de 72 %) est obtenu.

La pureté molaire estimée par RMN ¹H est supérieure à 86 %.

Protocole 19 (selon l'invention) :
Dans un réacteur de 1L, on charge 30 g de 3-hydroxy-2,4,6-triméthylbenzaldéhyde (0,18 mol) et 200 g de DMF. On ajoute 126,5 g de carbonate de potassium (0,92 mol) et on chauffe à reflux. On ajoute ensuite, par portions et sur une période de 6 heures, 67,9 g de 1-(2-chloroéthyl)imidazolidin-2-one (0,46 mol). A la fin de l'ajout, on laisse réagir pendant 2 heures à reflux. On refroidit à 50°C, puis on ajoute, sur une période de 15 minutes, 14,3 g d'une solution aqueuse d'hydroxylamine à 50% (0,22 mol). On laisse réagir pendant 2 heures à 50°C. Le mélange réactionnel est concentré jusqu'à un volume de 50 mL, puis on ajoute, à température ambiante, 500 mL d'eau. Le précipité obtenu est filtré, lavé à l'eau puis séché sous vide.

Un solide blanc-beige (44,6 g, rendement massique de 85%) avec un point de fusion de 167°C est obtenu.

Protocole 20 (selon l'invention) :
A un mélange de 3-hydroxy-2,4,6-triméthyl-benzaldéhyde (22,8 g, 0,139 mol) et de K₂CO₃ (115,2 g, 0,833 mol) dans l'isopropanol (160 mL) à 50°C, est ajoutée de la 1-(2-chloroéthyl)imidazolidin-2-one (30,9 g, 0,208 mol). La température du milieu réactionnel est portée à 100°C (T_{bain}) et maintenue pendant 3 à 4 heures. Ensuite, une seconde portion de 1-(2-chloroéthyl)imidazolidin-2-one (20,06 g, 0,139 mol, pureté technique) est ajoutée et le mélange est agité à 100°C (T_{bain}) pendant 5 heures. Enfin, la troisième portion de 1-(2-chloroéthyl)imidazolidin-2-one (20,06 g, 0,139 mol, pureté technique) est ajoutée et le mélange est agité à 100°C (Tbain) pendant 8 à 10 heures.

La température du milieu réactionnel est abaissée à température ambiante, puis de l'isopropanol (25 mL) est ajouté au milieu réactionnel. Le mélange est chauffé à 55°C et une solution d'hydroxylamine (14,68 g, 0,222 mol, 50 % dans l'eau, Aldrich) dans l'isopropanol (25 mL) est additionnée. Le milieu réactionnel est agité pendant 7 heures à 50°C. Le milieu réactionnel est versé dans de l'eau (3 L). Après agitation pendant 5 heures, le précipité obtenu est filtré, lavé sur le filtre par l'eau (600 mL) et séché à température ambiante.

Un solide gris clair (30,35 g, rendement massique de 75 %) est obtenu.

La pureté molaire estimée par RMN ¹H est supérieure à 87 %.

Protocole 21 (selon l'invention) :
Dans un réacteur de 1L, on charge 30 g de 3-hydroxy-2,4,6-triméthylbenzaldéhyde (0,18 mol) et 200 g d'isopropanol. On ajoute 126,5 g de carbonate de potassium (0,92 mol) et on chauffe à reflux. On ajoute ensuite, par portions et sur une période de 6 heures, 67,9 g de 1-(2-chloroéthyl)imidazolidin-2-one (0,46 mol). A la fin de l'ajout, on laisse réagir pendant 2 heures à reflux. On refroidit à 50°C, puis on ajoute, sur une période de 15 minutes, 14,3 g d'une solution aqueuse d'hydroxylamine à 50% (0,22 mol). On laisse réagir pendant 2 heures à 50°C. Le mélange réactionnel est concentré jusqu'à un volume de 50 mL, puis on ajoute, à température ambiante, 500 mL d'eau. Le précipité obtenu est filtré, lavé à l'eau puis séché sous vide.

Un solide blanc (43,0 g, rendement massique de 82%) avec un point de fusion de 167°C est obtenu.

### Etape d : préparation de l'oxyde de 2,4,6-triméthyl-3-[2-(2-oxoimidazolidin-1-yl)éthoxy]benzonitrile

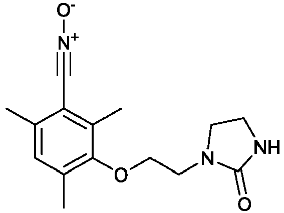

Protocole 22 :
Ce composé peut être obtenu selon un protocole décrit dans la demande de brevet WO 2012/007684.

Protocole 23 :
A un mélange d'oxime de 2,4,6-triméthyl-3-(2-(2-oxoimidazolidin-1-yl)éthoxy)benzaldéhyde (9,7 g, 0,033 mol) dans l'acétate d'éthyle (575 mL) à 1°C, est ajoutée, goutte à goutte, une solution aqueuse de NaOCl (48 % g Cl/L) (73 mL) pendant 10 minutes. La température du milieu réactionnel est maintenue entre 1 et 4°C. Le milieu réactionnel est agité pendant 1 heure et 30 minutes à 2-4°C. Le précipité obtenu est filtré, lavé sur le filtre par l'eau (trois fois par 20 mL) et par l'acétate d'éthyle (trois fois par 20 mL) et enfin séché pendant 10 à 15 heures sous pression atmosphérique à température ambiante.

Un solide blanc (8,63 g, rendement massique de 90 %) est obtenu.

La pureté molaire estimée par RMN ¹H est supérieure à 94 %.

Protocole 24 :
Dans un réacteur de 1L, on charge 40 g d'oxime de 2,4,6-triméthyl-3-(2-(2-oxoimidazolidin-1-yl)éthoxy)benzaldéhyde (0,14 mol) et 280 g d'acétate d'éthyle. On refroidit à 5°C et on ajoute, sur une période de 15 minutes, 127,5 g d'eau de Javel à 12% (0,17 mol). On laisse réagir pendant 3 heures à 5°C. On filtre et on lave le solide obtenu à l'eau puis à l'acétate d'éthyle.

Un solide blanc (33,7 g, rendement massique de 85 %) avec un point de fusion de 160°C est obtenu.

Ainsi, l'oxyde de 2,4,6-triméthyl-3-[2-(2-oxoimidazolidin-1-yl)éthoxy]benzonitrile est obtenu grâce à une synthèse monotope en deux étapes directement à partir du 3-hydroxy-2,4,6-triméthyl-benzaldéhyde, suivie d'une réaction d'oxydation.

Le procédé selon l'invention permet donc une amélioration du rendement global dudit procédé ainsi qu'une réduction des coûts d'investissement et de production.

### Exemple 3: Synthèse de l'oxyde de 3-méthoxy-4-(2-(2-oxoimidazolidin-1-yl)éthoxy)benzonitrile

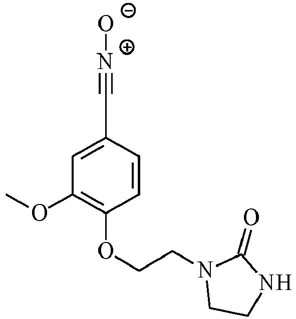

### Synthèse monotope en deux étapes b1 et b2 suivie de l'étape c (conforme à l'invention) : préparation de l'oxime de 3-méthoxy-4-(2-(2-oxoimidazolidin-1-yl)éthoxy) benzaldéhyde

Protocole 25 (selon l'invention) :
Dans un réacteur de 1L, on charge 30 g de vanilline (0,20 mol) et 200 g d'isopropanol. On ajoute 138,2 g de carbonate de potassium (1 mol) et on chauffe à reflux. On ajoute ensuite, par portions et sur une période de 6 heures, 89,2 g de 1-(2-chloroéthyl)imidazolidin-2-one (0,6 mol). A la fin de l'ajout, on laisse réagir pendant 2 heures à reflux. On refroidit à 50°C, puis on ajoute, sur une période de 15 minutes, 19,8 g d'une solution aqueuse d'hydroxylamine à 50% (0,30 mol). On laisse réagir pendant 2 heures à 50°C. Le mélange réactionnel est concentré jusqu'à un volume de 50 mL, puis on ajoute, à température ambiante, 500 mL d'eau. Le précipité obtenu est filtré, lavé à l'eau puis séché sous vide.

Un solide blanc (33,5 g, rendement massique de 60%) avec un point de fusion de 189°C est obtenu.

### Etape d : préparation de l'oxyde de 3-méthoxy-4-[2-(2-oxoimidazolidin-1-yl)éthoxy]benzonitrile

Protocole 26 :
Dans un réacteur de 1L, on charge 30 g d'oxime de 3-méthoxy-4-(2-(2-oxoimidazolidin-1-yl)éthoxy)benzaldéhyde (0,11 mol) et 300 g de dichlorométhane. On refroidit à 5°C et on ajoute, sur une période de 30 minutes, 102,4 g d'eau de Javel à 12% (0,17 mol). On laisse réagir pendant 3 heures à 5°C. On filtre et on lave le solide obtenu à l'eau puis au pentane.

Un solide blanc (15,5 g, rendement massique de 52%) avec un point de fusion de 111°C est obtenu.

## Revendications

1. Procédé de synthèse d'un composé de formule (I) suivante : 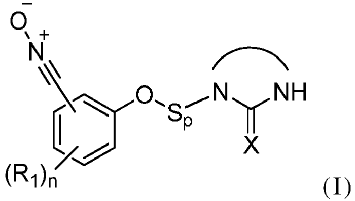 dans laquelle :
- X désigne un atome d'oxygène, de soufre ou un groupe NH, préférentiellement un atome d'oxygène ;
- R₁ représente un groupe alkyle en C₁-C₁₂ ou OR', R' étant un groupe alkyle en C₁-C₁₂ ;
- n est un nombre entier allant de 0 à 4 ;
- Sp représente une chaîne alkylène linéaire ou ramifiée en C₁-C24, éventuellement interrompue par un ou plusieurs atomes d'azote ou d'oxygène,
le groupe 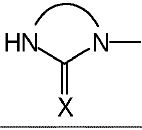 représentant un hétérocycle di ou triazoté, à 5 ou 6 atomes, de préférence diazoté, comprenant au moins une fonction carbonyle, thiocarbonyle ou imine.
comprenant deux étapes successives (b1) et (b2) selon une synthèse monotope :
- (b1) la réaction d'un composé de formule (II) : 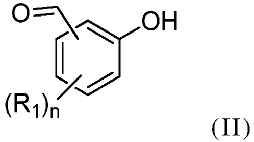
avec un composé de formule (III) : 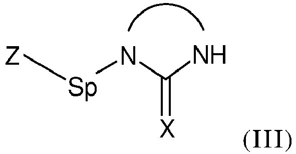 dans laquelle Z représente un groupe nucléofuge ;
en présence :
- d'au moins un solvant polaire S₁ ;
- d'au moins une base choisie parmi les carbonates alcalins ;
à une température T₁ allant de 70 à 150°C,
pour former un éther ;
- (b2) la réaction dudit éther avec une solution aqueuse d'hydroxylamine à une température T₂ allant de 30 à 70°C, pour obtenir un composé oxime de formule (IV) : 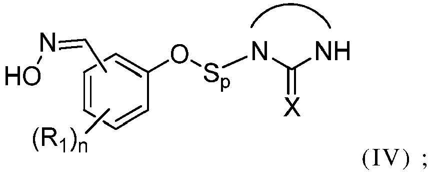 puis :
- une étape (c) de récupération dudit composé oxime de formule (IV) ;
- une étape (d) d'oxydation du composé oxime de formule (IV) avec un agent oxydant, en présence d'au moins un solvant organique S₂.

2. Procédé selon la revendication 1, dans lequel le groupe 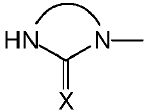 est le groupe 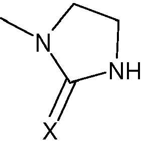 , dans lequel X est choisi parmi un atome d'oxygène et un atome de soufre, de préférence un atome d'oxygène.

3. Procédé selon l'une quelconque des revendications 1 à 2, dans lequel le groupe Sp est une chaîne alkylène linéaire ou ramifiée en C₁-C₁₀, éventuellement interrompue par un ou plusieurs atomes d'azote ou d'oxygène, plus préférentiellement une chaîne alkylène linéaire en C₁-C₆.

4. Procédé selon l'une quelconque des revendications précédentes, dans lequel le groupe Z est choisi parmi le chlore, le brome, l'iode, le groupe mésylate, le groupe tosylate, le groupe acétate et le groupe trifluorométhylsulfonate.

5. Procédé selon l'une quelconque des revendications précédentes, dans lequel le solvant polaire S₁ est un solvant polaire miscible dans l'eau, préférentiellement un solvant protique.

6. Procédé selon l'une quelconque des revendications précédentes, dans lequel la base est le carbonate de potassium.

7. Procédé selon l'une quelconque des revendications précédentes, dans lequel ledit agent oxydant est choisi parmi l'hypochlorite de sodium, le N-bromosuccinimide en présence d'une base, le N-chlorosuccinimide en présence d'une base, et l'eau oxygénée en présence d'un catalyseur, préférentiellement l'hypochlorite de sodium.

8. Procédé selon l'une quelconque des revendications précédentes, dans lequel le solvant organique S₂ est un solvant organique choisi parmi les solvants chlorés et de type ester, éther et alcool, plus préférentiellement choisi parmi le dichlorométhane, l'acétate d'éthyle, l'acétate de butyle, l'éther diéthylique, l'isopropanol et l'éthanol, encore plus préférentiellement choisi parmi l'acétate d'éthyle et l'acétate de butyle.

9. Procédé selon l'une quelconque des revendications précédentes, dans lequel le procédé comprend une étape (a1) de fabrication du composé de formule (II), préalable à l'étape (b1), par mise en réaction d'un composé de formule (VIII) : 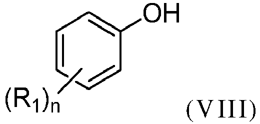 avec un agent de formylation formé in situ ou non et au moins un acide de Lewis, en présence d'au moins un solvant organique S₃.

10. Procédé selon la revendication précédente, dans lequel l'acide de Lewis est choisi parmi TiCl₄ et SnCl₄, préférentiellement TiCl₄.

11. Procédé selon l'une quelconque des revendications 9 à 10, dans lequel le solvant organique S₃ est un solvant organique chloré, préférentiellement choisi parmi le dichlorométhane, le chloroforme et le 1,2-dichloroéthane, plus préférentiellement le dichlorométhane.

12. Procédé selon l'une quelconque des revendications 9 à 11, dans lequel l'agent de formylation est choisi parmi le dichlorométhyl-méthyléther et le dichlorométhyl-éthyléther, préférentiellement le dichlorométhyl-méthyléther.

13. Procédé selon l'une quelconque des revendications précédentes, dans lequel le procédé comprend une étape (a2) de fabrication du composé de formule (III), préalable à l'étape (b1), par mise en réaction d'un composé de formule (IX) : 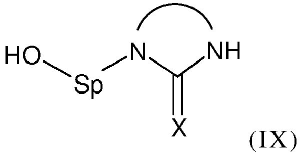 avec un agent permettant la formation du groupe nucléofuge Z.

14. Procédé selon la revendication précédente, dans lequel ledit agent est le chlorure de thionyle.

## Patentansprüche

1. Verfahren zur Synthese einer Verbindung der folgenden Formel (I): 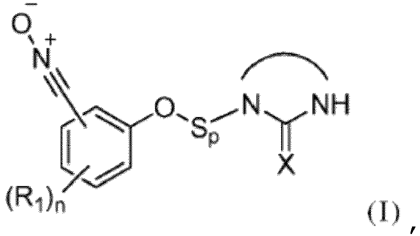 wobei:
- X ein Sauerstoff-, Schwefelatom oder eine NH-Gruppe, vorzugsweise ein Sauerstoffatom bezeichnet;
- R₁ für eine C₁-C₁₂-Alkylgruppe oder OR' steht, wobei R' eine C₁-C₁₂-Alkylgruppe ist;
- n eine ganze Zahl ist, die von 0 bis 4 reicht;
- Sp für eine lineare oder verzweigte C₁-C₂₄-Alkylengruppe steht, die gegebenenfalls durch ein oder mehrere Stickstoff- oder Sauerstoffatome unterbrochen ist,
wobei die Gruppe 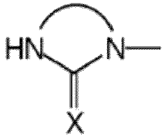 für einen Di- oder Tristickstoffheterocyclus mit 5 oder 6 Atomen, vorzugsweise einen Distickstoffheterocyclus steht, der mindestens eine Carbonyl-, Thiocarbonyl- oder Iminfunktion umfasst,
umfassend zwei aufeinanderfolgende Schritte (b1) und (b2) gemäß einer Eintopfsynthese:
- (b1) Reaktion einer Verbindung der Formel (II): 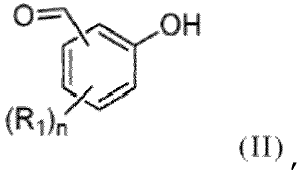
mit einer Verbindung der Formel (III): 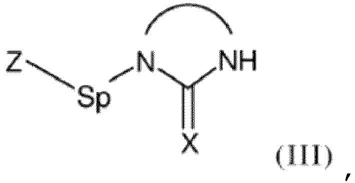 wobei Z für eine nukleofuge Gruppe steht;
in Gegenwart:
- von mindestens einem polaren Lösungsmittel S₁;
- von mindestens einer Base, die aus Alkalicarbonaten ausgewählt ist;
bei einer Temperatur T₁, die von 70 bis 150 °C reicht,
um einen Ether zu bilden;
- (b2) Reaktion des Ethers mit einer wässrigen Hydroxylaminlösung bei einer Temperatur T₂, die von 30 bis 70 °C reicht, um eine Oximverbindung der Formel (IV) zu erhalten: 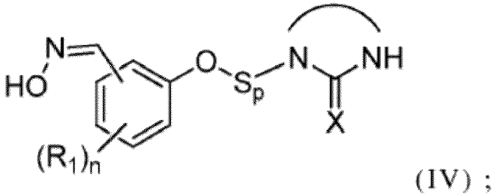 dann:
- einen Schritt (c) eines Gewinnens der Oximverbindung der Formel (IV);
- einen Schritt (d) eines Oxidierens der Oximverbindung der Formel (IV) mit einem Oxidationsmittel in Gegenwart von mindestens einem organischen Lösungsmittel S₂.

2. Verfahren nach Anspruch 1, wobei die Gruppe 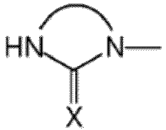 die Gruppe 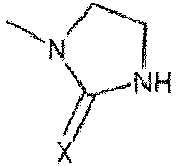 ist, wobei X aus einem Sauerstoffatom und einem Schwefelatom, vorzugsweise einem Sauerstoffatom ausgewählt ist.

3. Verfahren nach einem der Ansprüche 1 bis 2, wobei die Gruppe Sp eine lineare oder verzweigte C₁-C₁₀-Alkylenkette, die gegebenenfalls durch ein oder mehrere Stickstoff- oder Sauerstoffatome unterbrochen ist, mehr bevorzugt eine lineare C₁-C₆-Alkylenkette ist.

4. Verfahren nach einem der vorhergehenden Ansprüche, wobei die Gruppe Z aus Chlor, Brom, Iod, der Mesylatgruppe, der Tosylatgruppe, der Acetatgruppe und der Trifluormethylsulfonatgruppe ausgewählt ist.

5. Verfahren nach einem der vorhergehenden Ansprüche, wobei das polare Lösungsmittel S₁ ein polares Lösungsmittel, das mit Wasser mischbar ist, vorzugsweise ein protisches Lösungsmittel ist.

6. Verfahren nach einem der vorhergehenden Ansprüche, wobei die Base Kaliumcarbonat ist.

7. Verfahren nach einem der vorhergehenden Ansprüche, wobei das Oxidationsmittel aus Natriumhypochlorit, N-Bromsuccinimid in Gegenwart einer Base, N-Chlorsuccinimid in Gegenwart einer Base und Wasserstoffperoxid in Gegenwart eines Katalysators, vorzugsweise Natriumhypochlorit ausgewählt ist.

8. Verfahren nach einem der vorhergehenden Ansprüche, wobei das organische Lösungsmittel S₂ ein organisches Lösungsmittel ist, das aus chlorierten Lösungsmitteln und Lösungsmitteln vom Ester-, Ether- und Alkoholtyp ausgewählt ist, mehr bevorzugt aus Dichlormethan, Ethylacetat, Butylacetat, Diethylether, Isopropanol und Ethanol ausgewählt ist, noch mehr bevorzugt aus Ethylacetat und Butylacetat ausgewählt ist.

9. Verfahren nach einem der vorhergehenden Ansprüche, wobei das Verfahren einen Schritt (a1) eines Herstellens der Verbindung der Formel (II) vor dem Schritt (b1) durch Reagierenlassen einer Verbindung der Formel (VIII): 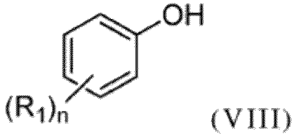 mit einem Formylierungsmittel, das in situ gebildet wird oder auch nicht, und mindestens einer Lewissäure in Gegenwart von mindestens einem organischen Lösungsmittel S₃ umfasst.

10. Verfahren nach dem vorhergehenden Anspruch, wobei die Lewissäure aus TiCl₄ und SnCl₄, vorzugsweise TiCl₄ ausgewählt ist.

11. Verfahren nach einem der Ansprüche 9 bis 10, wobei das organische Lösungsmittel S₃ ein chloriertes organisches Lösungsmittel ist, das vorzugsweise aus Dichlormethan, Chloroform und 1,2-Dichlorethan, mehr bevorzugt Dichlormethan ausgewählt ist.

12. Verfahren nach einem der Ansprüche 9 bis 11, wobei das Formylierungsmittel aus Dichlormethylmethylether und Dichlormethylethylether, vorzugsweise Dichlormethylmethylether ausgewählt ist.

13. Verfahren nach einem der vorhergehenden Ansprüche, wobei das Verfahren einen Schritt (a2) eines Herstellens der Verbindung der Formel (III) vor dem Schritt (b1) durch Reagierenlassen einer Verbindung der Formel (IX): 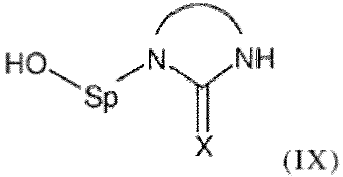 mit einem Mittel, das die Bildung der nukleofugen Gruppe Z ermöglicht, umfasst.

14. Verfahren nach dem vorhergehenden Anspruch, wobei das Mittel Thionylchlorid ist.

## Claims

1. A method for synthesising a compound of the following formula (I): 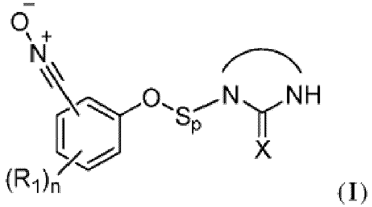 wherein
- X designates an oxygen, sulphur atom or an NH group, preferably an oxygen atom;
- R₁ represents a C₁-C₁₂ alkyl group or OR', R' being a C₁-C₁₂ alkyl group,
- n is an integer ranging from 0 to 4;
- Sp represents a linear or branched C₁-C₂₄ alkylene chain, optionally interrupted by one or more nitrogen or oxygen atoms,
the group 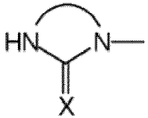 representing a 5 or 6-atom diazotized or triazotized, preferably diazotized, heterocycle, comprising at least one carbonyl, thiocarbonyl or imine function,
comprising two successive steps (b1) and (b2) according to a one-pot synthesis:
- (b1) reacting a compound of formula (II) 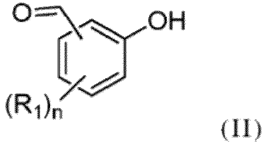 with a compound of formula (III): 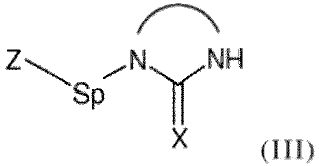
wherein Z represents a nucleofugal group; in the presence of:
- at least one polar solvent S1;
- at least one base selected from alkali carbonates;
at a temperature T₁ ranging from 70 to 150°C, to form an ether;
- (b2) reacting said ether with an aqueous hydroxylamine solution at a temperature T₂ ranging from 30 to 70°C, to obtain an oxime compound of formula (IV): 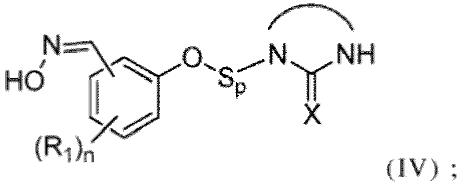 then:
- a step (c) of recovering said oxime compound of formula (IV) ;
- a step (d) of oxidising the oxime compound of formula (IV) with an oxidising agent, in the presence of at least one organic solvent S₂.

2. The method according to claim 1, wherein the group 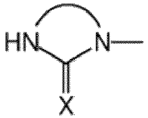 is the group 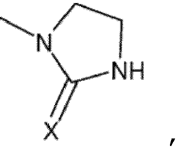 in which X is selected from an oxygen atom and a sulphur atom, preferably an oxygen atom.

3. The method according to any one of claims 1 to 2, wherein the group Sp is a linear or branched C₁-C₁₀ alkylene chain, optionally interrupted by one or more nitrogen or oxygen atoms, more preferably a linear C₁-C₆ alkylene chain.

4. The method according to any one of the preceding claims, wherein the group Z is selected from chlorine, bromine, iodine, the mesylate group, the tosylate group, the acetate group and the trifluoromethylsulphonate group.

5. The method according to any one of the preceding claims, wherein the polar solvent S₁ is a water-miscible polar solvent, preferably a protic solvent.

6. The method according to any one of the preceding claims, wherein the base is potassium carbonate.

7. The method according to any one of the preceding claims, wherein said oxidising agent is selected from sodium hypochlorite, N-bromosuccinimide in the presence of a base, N-chlorosuccinimide in the presence of a base, and hydrogen peroxide in the presence of a catalyst, preferably sodium hypochlorite.

8. The method according to any one of the preceding claims, wherein the organic solvent S₂ is an organic solvent selected from chlorinated solvents and of the ester, ether and alcohol type, more preferably selected from dichloromethane, ethyl acetate, butyl acetate, diethyl ether, isopropanol and ethanol, even more preferably selected from ethyl acetate and butyl acetate.

9. The method according to any one of the preceding claims, wherein the method comprises a step (a1) of manufacturing the compound of formula (II), prior to step (b1), by reacting a compound of formula (VIII): 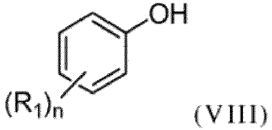 with a formylating agent formed in situ or not and at least one Lewis acid, in the presence of at least one organic solvent S₃.

10. The method according to the preceding claim, wherein the Lewis acid is selected from TiCl₄ and SnCl₄, preferably TiCl₄.

11. The method according to any one of claims 9 to 10, wherein the organic solvent S₃ is a chlorinated organic solvent, preferably selected from dichloromethane, chloroform and 1,2-dichloroethane, more preferably dichloromethane.

12. The method according to any one of claims 9 to 11, wherein the formylating agent is selected from dichloromethyl methyl ether and dichloromethyl ethyl ether, preferably dichloromethyl methyl ether.

13. The method according to any one of the preceding claims, wherein the method comprises a step (a2) of manufacturing the compound of formula (III), prior to step (b1), by reacting a compound of formula (IX): 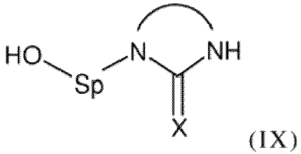 with an agent allowing the formation of the nucleofugal group.

14. The method according to the preceding claim, wherein said agent is thionyl chloride.
